# EUROPEAN PATENT SPECIFICATION

(11) **EP 2 285 447 B1**
(45) Date of publication and mention of the grant of the patent: **12.06.2013**
(21) Application number: 08749021.5
(22) Date of filing: 21.04.2008
(51) Int. Cl.: A61N 5/10

(54) **IMPROVEMENTS IN OR RELATING TO MULTI-LEAF COLLIMATORS**
VERBESSERUNGEN VON ODER IM ZUSAMMENHANG MIT MULTI-LEAF-KOLLIMATOREN
AMELIORATIONS CONCERNANT DES COLLIMATEURS MULTILAME

(43) Date of publication of application: 23.02.2011
(73) Proprietor: Elekta AB (publ), 103 93 Stockholm (SE)
(72) Inventor: BROAD, Martin, Kent BR2 7QF (GB)
(74) Representative: Hall, Christopher David
(86) International application number: PCT/EP2008/003183
(87) International publication number: WO 2009/129817

(56) References cited:
- EP-A- 0 193 509
- EP-A- 0 259 989
- WO-A-2007/124248

## Description

### FIELD OF THE INVENTION

The present invention relates to multi-leaf collimators.

### BACKGROUND ART

Radiotherapeutic apparatus involves the production of a beam of ionising radiation, usually x-rays or a beam of electrons or other sub-atomic particles. This is directed towards a cancerous region of the patient, and adversely affects the tumour cells causing an alleviation of the patient's symptoms. Generally, it is preferred to delimit the radiation beam so that the dose is maximised in the tumour cells and minimised in healthy cells of the patient, as this improves the efficiency of treatment and reduces the side effects suffered by a patient. A variety of methods of doing so have evolved.

One principal component in delimiting the radiation dose is the so-called "multi-leaf collimator" (MLC). This is a collimator which consists of a large number of elongate thin leaves arranged side to side in an array. Each leaf is moveable longitudinally so that its tip can be extended into or withdrawn from the radiation field. The array of leaf tips can thus be positioned so as to define a variable edge to the collimator. All the leaves can be withdrawn to open the radiation field, or all the leaves can be extended so as to close it down. Alternatively, some leaves can be withdrawn and some extended so as to define any desired shape, within operational limits. A multi-leaf collimator usually consists of two banks of such arrays, each bank projecting into the radiation field from opposite sides of the collimator.

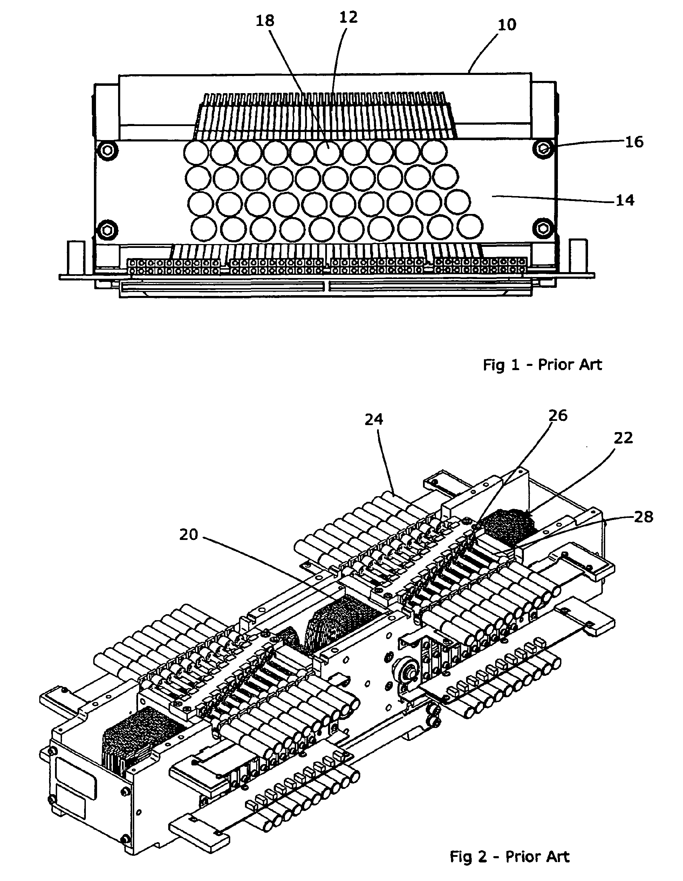

The leaves on the MLC leaf bank need to be driven in some way. Typically, this is by a series of lead screws connected to geared electric motors. The leaves are fitted with a small captive nut in which the lead screws fit, and the electric motors are fixed on a mounting plate directly behind the leaves. Rotation of the leadscrew by the motor therefore creates a linear movement of the leaf. The leaf drive motors are inevitably wider than a single leaf thickness, so in order to be able to drive each leaf the motors have to be mounted in a particular pattern as shown in figure 1. This shows a housing 10 for an array of adjacent MLC leaves 12. Behind the array, a motor mount 14 is fixed in place to housing 10 via bolts 16 so that it lies behind the leaves 12. A motor 18 for each leaf 12 is fixed to the motor mount 14.

Each motor 18 is generally tubular and from one end (as shown in figure 1) therefore appears circular. The motors are wider than an individual leaf and are therefore arranged in a staggered pattern. In this example, the motors 18 are arranged in four offset rows so that the centre of a motor is aligned with each leaf. As a result of this, the leadscrew nuts therefore have to be fixed to the leaves in one of a variety of positions, meaning that (in this case) four different leaf shapes need to be manufactured.

In an alternative system referred to as the "Beam Modulator" and shown generally in figure 2, leaves are driven by a rack and pinion system. A gear rack 20 is machined into the top or bottom of the leaves 22 and is driven by motors 24 fixed to the side of the leaf bank. The motor gear pinions 26 are mounted to an extension shaft 28 of a suitable length to enable the drive to be carried across to the appropriate leaf to be actuated.

In our earlier patent application GB-A-2423909, we describe a modular design similar to the Beam Modulator drive system. The application describes a design where a system of miniature gears and racks are incorporated into a detachable module. The linear motion is transmitted to the leaf via a slotted feature in the rack and engages in the leaf that is fitted with a 'tail'.

The choice of drive system is influenced by the quantity and thickness of the leaves in the leaf bank. For example, the MLC leaf bank has 40 leaves per side and has an average leaf thickness of 3.6mm. This thickness and quantity of leaves allows for a conventional solution of placing the motors directly behind the leaves and actuating them via a leadscrew which passes through the centre of the leaf.

The diameter of the leadscrew in this design is limited to 2.5mm, as this is largest diameter that can pass into the leaf without interfering with neighbouring leaves. Conveniently, it is also a standard ISO thread size. The leadscrew has to drive a leaf weighing around 800g, and at certain head/gantry angles the full weight of the leaf is suspended by the thread alone. Due to the small engagement area of the thread, the leadscrew therefore experiences high frictional loads and requires regular lubrication to maintain an acceptable service life. The performance of the leadscrew is also adversely affected by a whipping motion that can arise when the leaf nut is close to the motor, in which the long free end of the leadscrew can oscillate as it rotates. In addition, the leadscrew experiences a buckling load when the leaf is pushed to far end of the leadscrew. There is also a certain degree of noise due to this motion of the leadscrew.

The Beam Modulator design employs a thinner leaf in order to increase the resolution of the leafbank. This leaf thickness of only 1.75mm influences the selection of the drive system. A lead screw system as used on the MLC would not be a viable solution as it would require a 1.5mm diameter leadscrew; as the leaf travel is longer, the leadscrew would suffer increased whipping and buckling. Leadscrews with a high aspect ratio are also extremely difficult and costly to manufacture and are likely to fracture if they are not adequately supported. In addition, the quantity of motors required (40 per side) could not be fitted in behind the leaves due to their size.

The drive system for Beam Modulator therefore incorporates a rack and pinion system, with the motors disposed on either side, top and bottom of the leaf bank. The motors are fixed to the side of the leaf bank, and pinions are driven from the motors on extension shafts requiring 10 different lengths, in addition a staggered bearing block is incorporated in which the extension shafts runs. 8 such bearing blocks are required for the leaf bank.

Because the motors are dispersed along the 4 sides of the leaf bank, the bank has to be removed for motor servicing. Removal of the leaf bank is a lengthy process, and problems can occur with radiation performance if the leaf bank is not replaced in the same position.

The rack is machined into the top or bottom of the tungsten leaf; the bearing surface that would be positioned at the top of the leaf therefore has to be offset in order to make way for the rack. This has the undesired effect of reducing the shielding effect of the leaf, as some 8mm is lost off the top/bottom of the leaf for the rack and bearing surface.

In order for smooth operation of the rack a certain amount of clearance has to be maintained between the rack and pinion. Each of the 80 motors therefore has to be checked when assembling the leaf bank. This clearance can vary leaf to leaf, depending on manufacturing tolerances, and can lead to unwanted backlash once the pinion and motor gearbox begin to wear. Such backlash will affect the positional accuracy of the leaves.

GB-A-2423909 describes a removable module which alleviates many of the service issues problems experienced with the beam modulator. However, as it incorporates a rack and pinion system it will suffer from backlash in the same way. The MLC Rack and Pinion System was originally designed around a 160 leaf MLC, but limitations in available space in the Treatment Head above and below the leafbank as well as restrictions on the overall head diameter create problems for fitting this type of Actuator. The gear racks in the actuator are positioned to match the leaf pitch; during operation the racks extend into the radiation beam, which may have effects on beam performance - particularly if there is an error in the pitching. The Actuator module also contains a high parts count, including many precision cut gears and racks making this expensive to produce.

Thus, the leaf thickness/pitch and motor size affects the method in which the actuation is carried to the leaf, and once a suitable method is derived (of the 2 practical drive solutions, leadscrew and rack and pinion) the design can have inherent problems with wear, noise, production and assembly costs, backlash and servicing issues.

WO 2007/124248 discloses a multi-leaf collimator in which the leaves are designed to provide for high step resolution in the projected radiation energy shape. The motors are positioned behind each leaf. EP 0259989 discloses a multi-leaf collimator in which each leaf is driven by a gear motor via a flexible cable. EP 0193509 discloses a multi-leaf collimator in which low height, elongated, curved leaves are arranged side by side in opposed pairs. The configuration is such that the parts of the surfaces intersecting the irradiation field will always be directed generally towards the radiation source. Each leaf is driven by a motor via an upwardly directed extension, a threaded output shaft and a pivot pin.

### SUMMARY OF THE INVENTION

The present invention therefore seeks to provide a compact MLC actuator, that addresses many of the problems associated with a conventional leadscrew system, with the potential to drive a greater amount of leaves without relying on a complex drive design and a high parts count (relative to the number of leaves). This has the benefit of redudng production costs and assembly times. The drive mechanism should ideally not reduce the shielding effect of the tungsten leaves or interfere with the radiation beam. A modular design would also improve servicing issues by allowing the complete removal of the drive system from the leafbank.

The MLC actuator of the present invention is designed for use on a 160 leaf MLC, but can of course be applied to MLCs with a lesser or greater number of leaves. The drive will ideally be capable of moving the leaves faster than previous MLCs to offer better dynamic treatment therapies, and will be useable for MLCs with smaller width and/or pitch of the leaves of, say, 1.5 mm as compared to the 10mm diameter of the drive motors even within a limited overall head height.

The width above the leaves (i.e. on the source side) is generally smaller than that below the leaves, due to the tapered design of the leafbank. Therefore, any design should ideally encompass this difference in leaf width and available space without complicating the design and increasing the required numbers of component parts.

The present invention therefore provides a multi-leaf collimator as set out in the claims appended hereto.

Mounting the drive motors in this way allows them to be distributed more space-efficiently, and allows the drive system to be modular, without requiring rack and pinion gears.

To take advantage of the ability to distribute the motors in a more space-efficient manner, a multi-leaf collimator according to one aspect has a plurality of the motors mounted on the subframe at a first longitudinal end of the leadscrews, and the remainder of the motors mounted on the subframe are mounted at a second, opposing, longitudinal end of the leadscrews. Those leadscrews not at an edge of the array are preferably neighboured on either lateral side by one leadscrew driven by a motor mounted at the same longitudinal end and a second leadscrew driven by a motor mounted at the opposite longitudinal end. This results in the motors being arranged in pairs with a gap between which provides space for mounting the motors. The pairs of motors can be arranged one above the other to allow the necessary clearances, meaning that the leadscrews will be mounted in the subframe at one of two spacings from the leaf, with laterally neighbouring leadscrews being mounted at alternating spacings. The leadscrews can be mounted within a bore in the subframe.

Still greater space efficiency can be achieved in a second aspect of the invention by including a lower subframe, mounted at a location spaced from the leaf array in an opposite direction to that of the upper array and on which the remainder of the motors are mounted. This can be designed in a generally similar manner to that of the (upper) subframe, except as regards the leaf pitch which will need to be adjusted as a result of the varying inclination of the leaves. We prefer that half of the leaves are driven from the subframe and half are driven from the lower subframe. Adjacent leaves in the array can be driven alternately from the subframe and from the lower subframe.

The leaves are preferably mounted in a machined guide thereby to allow longitudinal motion. The subframe(s) can be mounted on the guide.

In this way, the leaves will be driven from an elongate edge thereof. This means that the leaves can comprise a front section of a first material that is substantially radiopaque, and a tail section via which they are driven.

The drive means can further include a threaded member on the leadscrew. This can urge a laterally extending lug, thereby to drive the leaf. The lug can engage with a recess on the leaf edge. It can be held in machined slot in the subframe; that slot can be machined with non-parallel sides to assist in guiding the lug in the light of the offset nature of the load that it needs to carry.

### BRIEF DESCRIPTION OF THE DRAWINGS

An embodiment of the present invention will now be described by way of example, with reference to the accompanying figures in which;
Figure 1 shows a view along the leaf direction of a known MLC drive arrangement;
Figure 2 shows a perspective view of a known beam modulator;
Figure 3 shows a single leaf according to the present invention;
Figure 4 shows a view of the leaf drive according to the present invention, along the direction of a leaf;
Figure 5 shows a bank of leaf drives according to the present invention;
Figure 6 shows the retention and removal of a single drive motor of the bank;
Figures 7 to 10 illustrate different profiles for the lug and the associated guide slot.

### DETAILED DESCRIPTION OF THE EMBODIMENTS

The inherent limitation on the minimum length of the rack and pinion-type system is the quantity of the motors mounted on the side of the module. For example, assuming that each module is designed to drive 40 leaves, that each motor is 10mm in diameter and (therefore) spaced 14mm apart in a double row, then the length of the module will have to be 14x(40/2), i.e. 280mm, plus the distance over which the leaves are expected to travel. If we take a rough figure of 70mm for this distance, this makes an overall length for the system of 350mm. The minimum overall height will be the motor diameter plus the height of the rack, i.e. about 32mm. A rack and pinion module when mounted on the leafbank will therefore increase the treatment head diameter significantly.

The MLC actuator described herein features a lead screw that runs parallel to the leaf, which means that the length of the drive modules are shorter overall, as the leadscrew only needs to be a slightly longer than the required leaf travel. The overall length of actuator including motors can therefore be about 200mm, with a height of about 24mm.

This however faces the difficulty noted above, i.e. that the leadscrew needs a minimum diameter in order to be economic to produce and sufficiently rigid in operation. For MLC arrays in which the individual leaf thickness falls close to or below this diameter, this raises difficulties in accommodating both the leadscrews and the motors that drive them.

The MLC actuator described herein incorporates a leadscrew drive assembly which actuates the leaf indirectly via a lug which projects out from the drive assembly and engages with a leaf. The leadscrews and lugs run in machined guide slots in a bearing block which both houses the lugs (etc) and provides mounting for the drive assemblies.

It still remains, of course, that the leadscrews may be wider than the leaves, and it will usually be the case that the motors are wider. Accordingly, each leaf will (generally) only be a fraction of the width of its associated drive mechanism. An alternative way of viewing this is that laterally arrayed drive mechanisms will only be able to drive a fraction of the leaves. Therefore, a number of such arrays can drive all of the leaves, if the drive from each array can be transmitted to the leaves satisfactorily. A specific pattern of drive mechanisms is therefore needed in order to mount the leadscrews drives into a compact removable module.

We have chosen to divide the drive to the leaves in a number of ways so as to distribute the drive mechanism arrays. First, leaves can be driven from their upper edge or their lower edge. This is defined by the convention that MLC arrays are usually described as having a top that is closest to the radiation source and a bottom that is closest to the patient. Such a convention is necessary since the MLC array is mounted in a radiation head that rotates around the patient, and therefore in use the array may take up any orientation. Thus, an upper subframe can carry half of the drive mechanisms and drive every other leaf, and a lower subframe can carry the other half to drive the remaining leaves. Next, each subframe can carry two rows of leadscrews, one above the other. The lugs associated with each leadscrew can be of a corresponding length. This spaces the motors and allows them to drive laterally adjacent leadscrews. Finally, the leadscrews do of course have two ends and can be driven from either. Accordingly, half the leadscrews in each subframe can be driven from the front (which we define as the end most distant from the beam) and half from the rear (defined correspondingly). Three such binary divisions allow 2³ combinations, i.e. each situationally identical drive means drive one in eight leaves. This division can be as follows:

| **Leaf** | **Subframe** | **Row** | **Bank** |
|---|---|---|---|
| 1* | Lower | bottom | front |
| 2 | Upper | top | front |
| 3 | Lower | top | front |
| 4 | Upper | bottom | front |
| 5 | Lower | bottom | rear |
| 6 | Upper | top | rear |
| 7 | Lower | top | rear |
| 8 | Upper | bottom | rear |
| 9* | Lower | bottom | front |
| 10 | Upper | top | front |
| 11 | Lower | top | front |
| 12 | Upper | bottom | front |
| 13 | Lower | bottom | rear |
| 14 | Upper | top | rear |
| 15 | Lower | top | rear |
| 16 | Upper | bottom | rear |
| 17* | Lower | bottom | front |
| 18 | Upper | top | front |
| 19 | Lower | top | front |
| 20 | Upper | bottom | front |
| 21 | Lower | bottom | rear |
| 22 | Upper | top | rear |
| 23 | Lower | top | rear |
| 24 | Upper | bottom | rear |
| 25* | Lower | bottom | front |
| 26 | Upper | top | front |
| 27 | Lower | top | front |
| 28 | Upper | bottom | front |
| 29 | Lower | bottom | rear |
| 30 | Upper | top | rear |
| 31 | Lower | top | rear |
| 32 | Upper | bottom | rear |

The precise pattern of the leadscrews, lugs, and guiding slots in the bearing block is derived from the angle and pitch of the leaf and the required space for the Drive motor. Such a pattern can also allow the drive motor axis to match the leaf centre line, ensuring an efficient transfer of linear motion.

By mounting the drive motors on the front and rear surfaces of the drive modules (upper and lower subframes) the area required to mount the drive motors can be dispersed over 2 faces. This also has the advantage of only requiring 2 sizes of drive mechanism, thereby maintaining a low parts count. Thus, the drive system is split into 2 modules; 2 per side, upper and lower. Each of these modules contains 40 motor/leadscrew drives, allowing for 80 leaves in total. Each module has 20 motors mounted on the front face and 20 on the rear face. The method for mounting of the motor/leadscrew drives is designed specifically to fit the pattern of machined slots in the modules.

This leadscrew design incorporates a precision machined leadscrew with an Acme thread form. The leadscrew nut is injection moulded in a low friction plastic material, which allows the assembly to run quietly without lubrication. The leadscrew nut fits into the lug, and can be easily replaced by removing the motor assembly.

The machined guide slots for the lugs can also be formed with non-parallel sides, and the lugs profiled correspondingly. Thus, viewed along the guide slot, the profile can be akin to that of a key for a cylinder lock. This provides non-vertical surfaces which act as bearings, removing from the leadscrew nut the side and moment loads which will occur in moving the mass of the tungsten leaf. On previous designs, this adversely affects the life of the nut. The leadscrew is also supported in this way, reducing both whipping and buckling tendencies. The guide slot profile may also feature a "V" or fir tree shape in the leg of the slot, which will increase the bearing surface area of the key and reduce friction.

A lower portion of the lugs are exposed below the drive module. These sections engage into the top or bottom of the tungsten leaf via a mating cut-out in the leaf. As this is small and in a part of the leaf that is not active in shaping the beam, the shielding performance of the MLC is not affected.

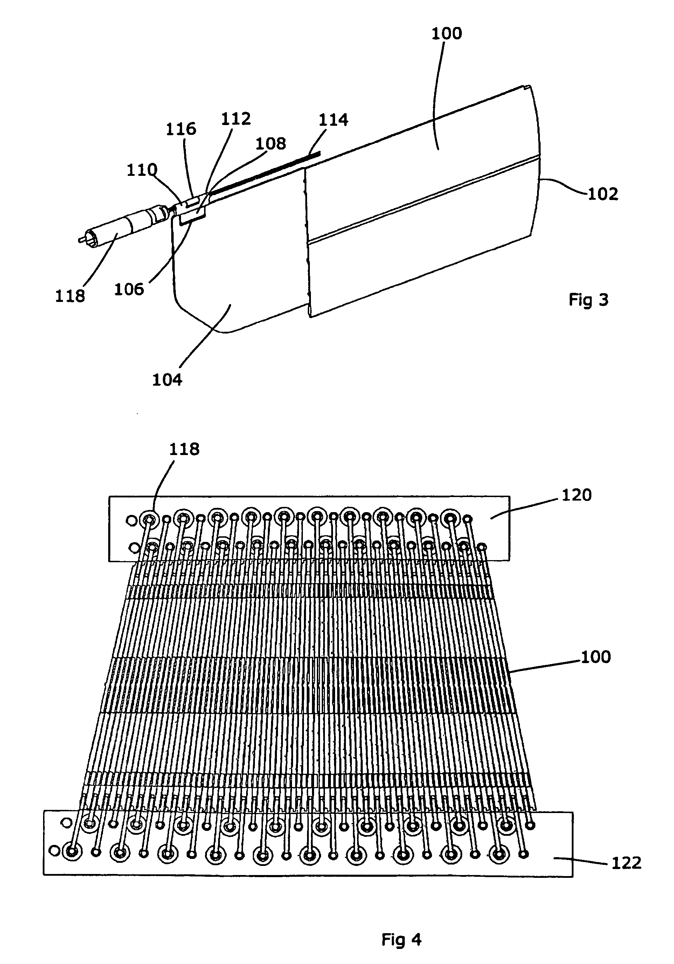

Referring to figure 3, this shows a single leaf and its associated drive. The tungsten attenuation portion 100 is relatively thin in a lateral direction in order to allow good resolution, is long in its longitudinal direction to allow a wide range of movement, and is deep in the beam direction to allow good attenuation of the beam. A front edge 102 of the attenuation portion 100 is curved in a generally known manner so as to provide a sharper penumbra. A rear edge of the attenuation portion 100 is vertical, and is joined to a drive portion 104 which makes up the remainder of the leaf.

The drive portion 104 has one edge, in this case the upper edge, which is co-linear with the corresponding edge of the attenuation portion 100 except for a recess 106 into which a lug 108 fits snugly. The opposing edge of the drive portion 104 is rebated back from the corresponding edge of the attenuation portion 100 in order to reduce the overall weight of the device and to avoid interference with the drive mechanism on the other side. It will be apparent that the relative orientations of the attenuation and drive portions can be reversed to allow the leaf to be driven from the top edge (as shown) or from the bottom edge.

The lug 108 fits snugly in the recess 106 of the drive portion 104 but is not fixed in place. The lug 108 is however attached to a pair of cylinders 110, 112 through which a leadscrew 114 passes, and between which a leadscrew nut 116 is fixed. Thus, as the leadscrew 114 is rotated, the nut 116 is forced in one direction or another and takes with it the cylinders 110, 112, the lug 108, the drive portion 104 and the attenuation portion 100. The cylinders offer rigidity to the structure retaining the leadscrew nut 116, and also offer lateral support to the leadscrew 144 to inhibit both whipping and buckling.

Finally, at one end of the leadscrew 114, a motor 118 is provided in order to drive the leadscrew.

Thus, by simple reversal of the orientations of the drive portion 104 and/or the motor 118/leadscrew 114, two of the above divisions can be achieved. The remaining third division is achieved by substitution of a longer lug 108. Accordingly, the spatial distribution of the various drive motors is achieved with an exceptionally low parts count.

Figure 4 shows one leaf bank from one end. The side-by side (i.e. laterally arrayed) leaves 100 are supported at their top and bottom edges in a leaf guide (not visible). Counting the leaves from the left hand side of figure 4, the odd-numbered leaves are driven from their lower edge and the even-numbered leaves are driven from their upper edge. Thus, an upper subframe 120 carries leadscrews, lugs, motors etc for the even-numbered leaves and a lower subframe 122 carries leadscrews, lugs, motors etc for the odd-numbered leaves. Apart from dimensional issues relating to the divergent nature of the leaves 100, the two subframes are functionally and structurally identical.

Within each subframe, for example the upper subframe 120, the first two leaves that are controlled (i.e. leaves 2 and 4) are connected via lugs 108 of varying lengths to a leadscrew running in a guide machined in the otherwise solid block that forms the subframe. These two guides are placed at differing heights so as to separate the motors 118.

The next leaf (i.e. leaf 6) is then connected to a leadscrew at the same upper level as leaf 2. To provide sufficient space, the motor for leaf 6 is located at the other end of the subframe 120 and drives its associated leadscrew from its other end. The pattern then continues, so that the next leaf that is driven in a manner identical to leaf 2 is leaf 10.

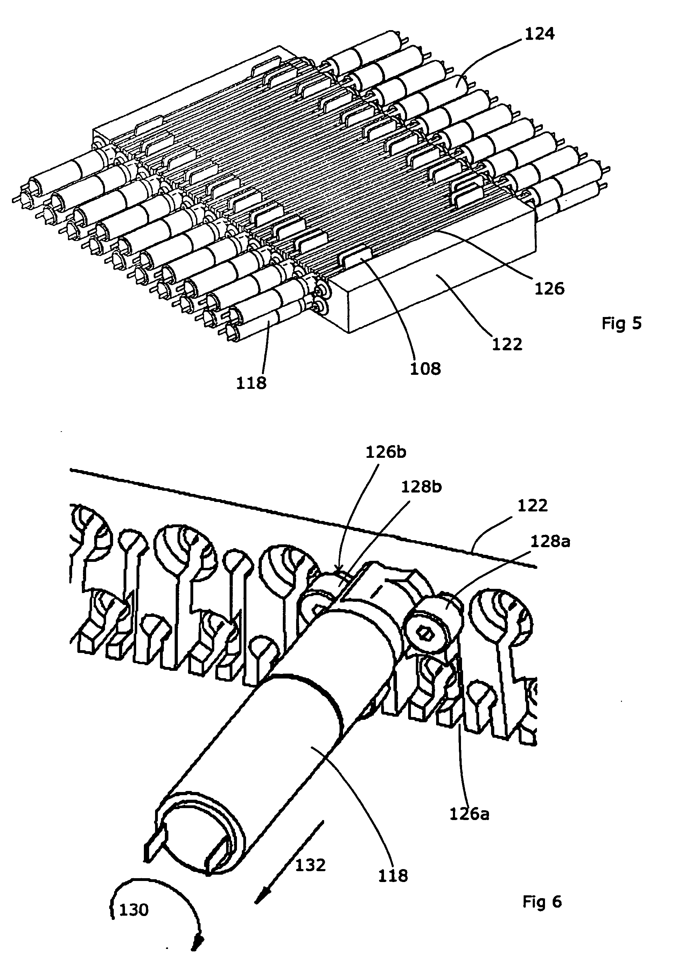

Figure 5 shows one subframe, with the leaf bank and leaf guide removed. An array of motors 118 can be seen at one end, distant from the beam, and an opposing array of motors 124 can be seen at the other end, closest to the beam. The lugs 108 can be seen projecting from the guide slots 126; when this subassembly is replaced under (or over) the leaf array then these lugs will project into the recesses 106 of the drive portions 104 of the leaves 100. In this way, the drive mechanism can be easily removed for service, repair or replacement.

Figure 6 shows how the motors 118 are retained on the subframe 122. Each motor has a pair of flanges projecting outwardly in two opposed directions around a part (but not all) of the circumference of the motor 118. Fortuitously, there will be a pair of guide slots 126a and 126b either side of the motor 118 which contain a leadscrew that is driven from the other end of the subframe 122. Thus, the ends (at least) of these slots 126a and 126b will be empty, and thus a mushroom-head screw 128a and 128b respectively can be screwed into the end of these slots 126a and 126b by providing a suitable tapping in the ends of the slots. In this way, by rotating the motor 118 so that the flanges are located under the mushroom-headed screws, then tightening the screws, the motor 118 will be retained securely. To remove the motor 118, both screws can be loosened, and the motor rotated in the direction of arrow 130 to move the flanges clear of the screw heads and allow the motor to be withdrawn in the direction of arrow 132.

In this arrangement, each screw will retain two motors, one on either side. This still permits individual motors to be removed, since the motors either side will still be retained by one screw, on their other side. This is generally preferable to providing each motor with a single flange and a single retaining screw; whilst this could be done, and would mean that each screw only held one motor, it would weaken the retention of the motors generally.

There could of course be further layers of leadscrews and motors beyond the two illustrated. Although this will incur a cost in terms of a greater complexity, it will permit a still greater ratio of motor spacing to leaf thickness to be achieved.

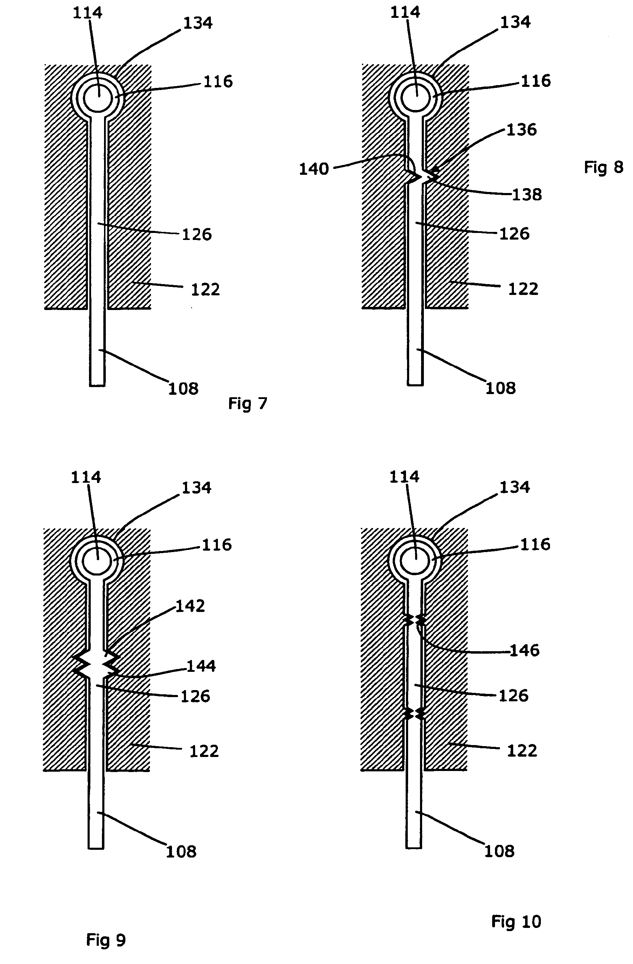

Figures 7 to 10 show alternative profiles for the lug 108 and the guide slot 126 in which it slides. Figure 7 shows the simplest option, a parallel-sided guide slot 126 formed in the subframe 122, with an enlarged root 134. The leadscrew 114 sits in the enlarged root 134 and is surrounded by the leadscrew nut 116. The lug 108 extends from the leadscrew nut 116, along the guide slot 126 and out of the subframe 122, to engage with the drive portion 104 of the leaf 100. This arrangement is obviously easiest to manufacture. However, it then requires the lug 108 to support the leaf 100 despite the fact that the centre of mass of the leaf 100 is offset from the line along which the lug 108 is driven. This will create a rotational moment on the lug 108 which will seek to rotate the lug 108 within the plane of the guide slot 126. This will create an uneven wear pattern on the lug 108, the leadscrew nut 116, and the leadscrew 114 and may be detrimental to the long-term performance of the drive mechanism.

Figure 8 therefore shows an adjustment to this design to alleviate this. The lug 108 is no longer parallel-sided, but includes a step 136 to one side part way along its length. The thickness of the lug 108 remains the same through the step; that is, the outward bulge 138 on one side is matched by a corresponding recess 140 on the other side. Matching formations are provided in the guide slot 126, to accommodate the outward bulge and to project into the recess.

By providing a non-flat surface to the lug 108 and a corresponding shape to the guide slot 126, rotation of the lug 108 in the guide slot 126 is inhibited. Support for the lug 108 against rotation is provided by the interaction of the bulge 138 and the recess 140 with the corresponding formations in the guide slot 126. Some lubrication may be useful in these areas, and a coating of graphite is suitable.

The arrangement shown in figure 8 is a simple and straightforward one which illustrates the concept. In practice, the bulges and recesses could be located elsewhere along the height of the lug 108/guide slot 126, and/or they could be duplicated so that multiple such formations are present. Where several such formations are provided, they could be oriented in the same direction, or in different orientations such as alternate directions or a mix of directions.

Figure 9 shows a further variation. In this arrangement, the lug 108 has a pair of adjacent bulges 142, 144 on one side, duplicated on the other side. Corresponding recesses are formed in the guide slot 126. This arrangement has the advantage of being symmetrical as compared to that of figure 8, and also avoids any narrowing of the lug 108 that might cause it to be weakened.

Figure 10 shows a further alternative. A pattern of recesses 146 are formed in the sides of the lug 108, in this case four on each side in two groups of two each. Corresponding bulges are provided on the internal surfaces of the guide slot 126.

The shapes described above can be formed at the necessary scale by processes such as wire discharge machining.

It will of course be understood that many variations may be made to the above-described embodiment without departing from the scope of the present invention.

## Claims

1. A multi-leaf collimator for a radiotherapy apparatus, comprising at least one array of laterally-spaced elongate leaves (100), each leaf being driven by an associated motor (118) connected to the leaf via a drive means so as to extend or retract the leaf in its longitudinal direction, the drive means for an array of the at least one array comprising a sub-frame (120) on which at least a subset of the motors are mounted, the sub-frame being mounted at a location spaced from the leaf array in a direction transverse to the lateral and longitudinal directions, and including a plurality of leadscrews (114) disposed longitudinally, each being driven by a motor and being operatively connected to a leaf thereby to drive that leaf, **characterised by** a plurality of the motors mounted on the sub-frame being mounted at a first longitudinal end of the leadscrews and the remainder being mounted at a second, opposing, longitudinal end of the leadscrews.

2. A multi-leaf collimator according to claim 1, wherein the subframe is an upper subframe, and further comprising a lower subframe (122) mounted at a location spaced from the leaf array in an opposite direction to that of the upper subframe and on which the remainder of the motors are mounted.

3. A multi-leaf collimator for a radiotherapy apparatus, comprising at least one array of laterally-spaced elongate leaves (100), each leaf being driven by an associated motor (118) connected to the leaf via a drive means so as to extend or retract the leaf in its longitudinal direction, the drive means comprising a sub-frame (120) on which at least a subset of the motors are mounted, the sub-frame being mounted at a location spaced from the leaf array in a direction transverse to the lateral and longitudinal directions, and including a plurality of leadscrews (114) disposed longitudinally, each being driven by a motor and being operatively connected to a leaf thereby to drive that leaf, wherein the subframe is an upper subframe, **characterised by** further comprising a lower subframe (122) mounted at a location spaced from the leaf array in an opposite direction to that of the upper subframe and on which the remainder of the motors are mounted.

4. A multi-leaf collimator according to claim 3 in which a plurality of the motors mounted on the subframe are mounted at a first longitudinal end of the leadscrews and the remainder are mounted at a second, opposing, longitudinal end of the leadscrews.

5. A multi-leaf collimator according to claim 1, 2 or 4 in which the leadscrews are nelghboured on either lateral side by one leadscrew driven by a motor mounted at the same longitudinal end and a second leadscrew driven by a motor mounted at the opposite longitudinal end.

6. A multl-leaf collimator according to any one of the preceding claims In which the leadscrews are mounted in the subframe at one of two spacings from the leaf, laterally neighbouring leadscrews being mounted at alternating spacings.

7. A multi-leaf collimator according to any one of the preceding claims in which the leadscrews are mounted within a bore In the subframe.

8. A multi-leaf collimator according to any one of claims 2 to 7 in which half of the leaves are driven from the upper subframe and half are driven from the lower subframe.

9. A multi-leaf collimator according to claim 8 in which adjacent leaves in the array are driven alternately from the upper subframe and from the lower subframe.

10. A multi-leaf collimator according to any one of the preceding claims in which the leaves are mounted in a machined guide thereby to allow longitudinal motion.

11. A multi-leaf collimator according to claim 10 in which the subframe is mounted on the guide.

12. A multi-leaf collimator according to claim 10 as dependent on any of claims 2 to 9 in which the lower subframe is mounted on the guide.

13. A multi-leaf collimator according to any one of the preceding claims in which the leaves are driven from an elongate edge thereof.

14. A multi-leaf collimator according to claim 13 in which the leaves comprise a front section of a first material that is substantially radiopaque and a tall section via which they are driven.

15. A multi-leaf collimator according to any one of the preceding claims in which the drive means further includes a threaded member on the leadscrew.

16. A multi-leaf collimator according to claim 15 in which the threaded member urges a laterally extending lug thereby to drive the leaf.

17. A multi-leaf collimator according to claim 16 in which the lug engages with a recess on the leaf edge.

18. A multi-leaf collimator according to claim 16 or claim 17 in which the lug is held in machined slot in the subframe.

19. A multi-leaf collimator according to claim 18 in which the slot has non-parallel sides.

## Patentansprüche

1. Multilamellenkollimator für eine Strahlentherapievorrichtung mit wenigstens einer Anordnung von seitlich beabstandeten, länglichen Lamellen (100), wobei jede Lamelle durch einen zugeordneten Motor (118) angetrieben wird, der mit der Lamelle über eine Antriebseinrichtung verbunden ist, um so die Lamelle in ihrer Längsrichtung vorzuschieben oder zurückzuziehen, wobei die Antriebseinrichtung für eine Anordnung der wenigstens einen Anordnung ein Unterbauteil (120) aufweist, an dem wenigstens eine Teilmenge der Motoren angebracht ist, wobei das Unterbauteil an einem Ort entfernt von der Lamellenanordnung in einer Richtung quer zu den Seiten- und Längsrichtungen montiert ist und eine Mehrzahl von in Längsrichtung angeordneten Spindeln (114) umfasst, von denen jede durch einen Motor angetrieben und funktionsmäßig mit einer Lamelle verbunden ist, um dadurch diese Lamelle anzutreiben, **dadurch gekennzeichnet, dass** mehrere der an dem Unterbauteil montierten Motoren an einem ersten Längsende der Spindeln und die übrigen Motoren an einem zweiten, gegenüberliegenden Längsende der Spindeln montiert sind.

2. Multilamellenkollimator nach Anspruch 1, wobei das Unterbauteil ein oberes Unterbauteil ist und weiterhin ein unteres Unterbauteil (122) aufweist, das an einem Ort auf Abstand zu der Lamellenanordnung in entgegengesetzter Richtung gegenüber derjenigen des oberen Unterbauteils montiert ist und auf dem die übrigen Motoren angebracht sind.

3. Multilamellenkollimator für eine Strahlentherapievorrichtung mit wenigstens einer Anordnung von seitlich beabstandeten länglichen Lamellen (100), wobei jede Lamelle durch einen zugeordneten Motor (118) angetrieben wird, der über eine Antriebseinrichtung mit der Lamelle verbunden ist, um die Lamelle in ihrer Längsrichtung vorzuschieben oder zurückzuziehen, wobei die Antriebseinrichtung ein Unterbauteil (120) aufweist, an dem wenigstens eine Teilmenge der Motoren angebracht ist, wobei das Unterbauteil an einem Ort entfernt von der Lamellenanordnung in einer Richtung quer zu den Seiten- und Längsrichtungen montiert ist und eine Mehrzahl von in Längsrichtung angeordneten Spindeln (114) umfasst, von denen jede durch einen Motor angetrieben wird und funktionsmäßig mit einer Lamelle verbunden ist, um dadurch diese Lamelle anzutreiben, wobei das Unterbauteil ein oberes Unterbauteil ist, **dadurch gekennzeichnet, dass** ferner ein unteres Unterbauteil (122) vorhanden ist, das an einem Ort entfernt von der Lamellenanordnung in entgegengesetzter Richtung zu dem oberen Unterbauteil angebracht ist und an dem die übrigen der Motoren angebracht sind.

4. Multilamellenkollimator nach Anspruch 3, bei dem mehrere der an dem Unterbauteil angebrachten Motoren an einem ersten Längsende der Spindeln und die obrigen an einem zweiten, gegenüberliegenden Längsende der Spindeln angebracht sind.

5. Multilamellenkollimator nach Anspruch 1, 2 oder 4, bei dem die Spindeln auf beiden Seiten benachbart sind durch eine Spindel, die von einem an dem gleichen Längsende angebrachten Motor angetrieben wird, und durch eine zweite Spindel, die von einem Motor angetrieben wird, der am gegenüberliegenden Längsende angebracht ist.

6. Multilamellenkollimator nach einem der vorhergehenden Ansprüche, bei dem die Spindeln in dem Unterbauteil an einem von zwei Abständen von der Lamelle angebracht sind, wobei seitlich benachbarte Spindeln in abwechselnden Abständen angebracht sind.

7. Multilamellenkollimator nach einem der vorhergehenden Ansprüche, bei dem die Spindeln innerhalb einer Bohrung in dem Unterbauteil angebracht sind.

8. Multilamellenkollimator nach einem der Ansprüche 2 bis 7, bei dem die Hälfte der Lamellen von dem oberen Unterbauteil und die Hälfte von dem unteren Unterbauteil aus angetrieben werden.

9. Multilamellenkollimator nach Anspruch 8, bei dem benachbarte Lamellen in der Anordnung abwechselnd von dem oberen Unterbauteil und dem unteren Unterbauteil angetrieben werden.

10. Multilamellenkollimator nach einem der vorhergehenden Ansprüche, bei dem die Lamellen in einer bearbeiteten Führung angebracht sind, um dadurch Längsbewegungen zu erlauben.

11. Multilamellenkollimator nach Anspruch 10, bei dem das Unterbauteil an der Führung angebracht ist.

12. Multilamellenkollimator nach Anspruch 10, wenn abhängig von einem der Ansprüche 2 bis 9, bei dem das untere Unterbauteil an der Führung angebracht ist.

13. Multilamellenkollimator nach einem der vorhergehenden Ansprüche, bei dem die Lamellen von einer ihrer Längskanten aus angetrieben werden.

14. Multilamellenkollimator nach Anspruch 13, bei dem die Lamellen eine Vorderabschnitt aus einem ersten Material, das im Wesentlichen strahlungsundurchlässig ist, und einen hinteren Abschnitt aufweist, über den sie angetrieben werden.

15. Multilamellenkollimator nach einem der vorhergehenden Ansprüche, bei dem die Antriebseinrichtung weiter ein Gewindeteil auf der Spindel umfasst.

16. Multilamellenkollimator nach Anspruch 15, bei dem das Gewindeteil einen seitlich vorstehenden Fortsatz antreibt, um dadurch die Lamelle anzutreiben.

17. Multilamellenkollimator nach Anspruch 16, bei dem der Fortsatz in eine Vertiefung an der Lamellenkante eingreift.

18. Multilamellenkollimator nach Anspruch 16 oder Anspruch 17, bei dem der Fortsatz in einem eingearbeiteten Schlitz in dem Unterbauteil gehalten ist.

19. Multilamellenkollimator nach Anspruch 18, bei dem der Schlitz nicht-parallele Seiten hat.

## Revendications

1. Collimateur multilame pour appareil de radiothérapie, comprenant au moins un ensemble de lames allongées espacées latéralement (100), chaque lame étant entraînée par un moteur associé (118), connecté à la lame par l'intermédiaire d'un moyen d'entraînement afin d'étendre ou de rétracter la lame dans sa direction longitudinale, le moyen d'entraînement pour un ensemble desdits au moins un ensemble comprenant un faux cadre (120) sur lequel sont montés au moins un sous-ensemble des moteurs, le faux cadre étant monté en un emplacement espacé de l'ensemble de lames dans une direction transversale aux directions latérale et longitudinale, et comprenant une pluralité de vis mères (114) disposées longitudinalement, chacune étant entraînée par un moteur et étant connectée fonctionnellement à une lame afin d'entraîner cette lame, **caractérisé en ce qu'**une pluralité des moteurs montés sur le faux cadre sont montés à une première extrémité longitudinale des vis mères et le reste est monté en une seconde extrémité longitudinale, opposée, des vis mères.

2. Collimateur multilame selon la revendication 1, dans lequel le faux cadre est un faux cadre supérieur, et comprenant en outre un faux cadre inférieur (122) monté en un emplacement espacé de l'ensemble de lames dans une direction opposée à celle du faux cadre supérieur et sur lequel est monté le reste des moteurs.

3. Collimateur multilame pour appareil de radiothérapie, comprenant au moins un ensemble de lames allongées espacées latéralement (100), chaque lame étant entraînée par un moteur associé (118), connecté à la lame par l'intermédiaire d'un moyen d'entraînement afin d'étendre ou de rétracter la lame dans sa direction longitudinale, le moyen d'entraînement comprenant un faux cadre (120) sur lequel sont montés au moins un sous-ensemble des moteurs, le faux cadre étant monté en un emplacement espacé de l'ensemble de lames dans une direction transversale aux directions latérale et longitudinale, et comprenant une pluralité de vis mères (114) disposées longitudinalement, chacune étant entraînée par un moteur et étant connectée fonctionnellement à une lame afin d'entraîner cette lame, dans lequel le faux cadre est un faux cadre supérieur, **caractérisé en ce qu'**il comprend en outre un faux cadre inférieur (122) monté en un emplacement espacé de l'ensemble de lames dans une direction opposée à celle du faux cadre supérieur et sur lequel est monté le reste des moteurs.

4. Collimateur multilame selon la revendication 3, dans lequel une pluralité des moteurs montés sur le faux cadre sont montés en une première extrémité longitudinale des vis mères et le reste est monté en une seconde extrémité longitudinale opposée des vis mères.

5. Collimateur multilame selon la revendication 1, 2 ou 4, dans lequel les vis mères sont encadrées sur chaque côté latéral par une vis mère entraînée par un moteur monté sur la même extrémité longitudinale et une deuxième vis mère entraînée par un moteur monté sur l'extrémité longitudinale opposée.

6. Collimateur multilame selon l'une quelconque des revendications précédentes, dans lequel les vis mères sont montées dans le faux cadre à un ou deux espacements de la lame, les vis mères qui encadrent latéralement étant montées en des espacements alternés.

7. Collimateur multilame selon l'une quelconque des revendications précédentes, dans lequel les vis mères sont montées dans un alésage réalisé dans le faux cadre.

8. Collimateur multilame selon l'une quelconque des revendications 2 à 7, dans lequel la moitié des lames est entraînée depuis le faux cadre supérieur et la moitié est entraînée depuis le faux cadre inférieur.

9. Collimateur multilame selon la revendication 8, dans lequel les lames adjacentes de l'ensemble sont entraînées alternativement depuis le faux cadre supérieur et depuis le faux cadre inférieur.

10. Collimateur multilame selon l'une quelconque des revendications précédentes, dans lequel les lames sont montées dans un guide usiné afin de permettre un mouvement longitudinal.

11. Collimateur multilame selon la revendication 10, dans lequel le faux cadre est monté sur le guide.

12. Collimateur multilame selon la revendication 10 lorsqu'elle dépend de l'une quelconque des revendications 2 à 9, dans lequel le faux cadre inférieur est monté sur le guide.

13. Collimateur multilame selon l'une quelconque des revendications précédentes, dans lequel les lames sont entraînées à partir d'un de leurs bords allongés.

14. Collimateur multilame selon la revendication 13, dans lequel les lames comprennent une section avant faite d'un premier matériau qui est substantiellement radio-opaque et une section arrière par laquelle elles sont entraînées.

15. Collimateur multilame selon l'une quelconque des revendications précédentes, dans lequel le moyen d'entraînement comprend en outre un élément fileté sur la vis mère.

16. Collimateur multilame selon la revendication 15, dans lequel l'élément fileté pousse une oreille s'étendant latéralement afin d'entraîner la lame.

17. Collimateur multilame selon la revendication 16, dans lequel l'oreille s'engage dans un évidement prévu sur le bord de la lame.

18. Collimateur multilame selon la revendication 16 ou 17, dans lequel l'oreille est maintenue dans une fente usinée dans le faux cadre.

19. Collimateur multilame selon la revendication 18, dans lequel la fente a des côtés non parallèles.
